# EUROPEAN PATENT APPLICATION

(11) **EP 4 512 872 A1**
(43) Date of publication of application: **26.02.2025**
(21) Application number: 23791569.9
(22) Date of filing: 10.03.2023
(51) Int. Cl.: C09K 23/54, A61K 8/894, A61Q 1/00, A61Q 1/04, A61Q 1/10, A61Q 1/14, A61Q 17/04, A61Q 19/00, C08G 77/46

(54) **SURFACTANT AND COSMETIC**

(30) Priority: 19.04.2022 JP 2022068780
(71) Applicant: SHIN-ETSU CHEMICAL CO., LTD., Tokyo 1000005 (JP)
(72) Inventor: HATA Ryunosuke, Annaka-shi, Gunma 379-0224 (JP)
(74) Representative: Sonnenhauser, Thomas Martin
(86) International application number: PCT/JP2023/009315
(87) International publication number: WO 2023/203916

(57) **Abstract**

The present invention is a surfactant represented by the following general formula (1), 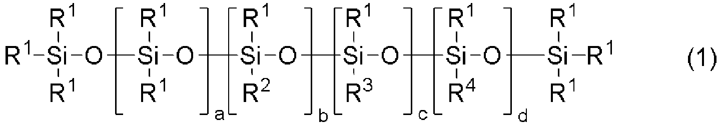 wherein R¹ independently represents a group selected from an alkyl group and an aryl group, R² independently represents a group represented by the following general formula (2), R³ independently represents an alkyl group, R⁴ independently represents a group having a cyclic siloxane structure represented by the following general formula (3), a = 10 to 60, b = 1 to 30, c = 0 to 30, and d = 1 to 30, 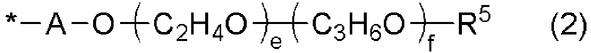 wherein A represents an alkylene group having 2 to 10 carbon atoms, R⁵ represents a hydrogen atom or an alkyl group, and "*" represents an attachment bonded to a silicon atom, 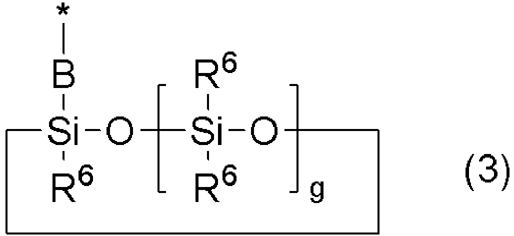 wherein B represents an alkylene group, R⁶ independently represents a hydrogen atom or a group selected from an alkyl group and an aryl group, "g" represents an integer satisfying g = 2 to 5, and "*" represents an attachment bonded to a silicon atom.

This provides a surfactant having a high effect of reducing interfacial tension.

## Description

### TECHNICAL FIELD

The present invention relates to a surfactant and a cosmetic.

### BACKGROUND ART

As an oily component in a cosmetic, silicone oil is commonly used due to its good feel of use. When the cosmetic using the silicone oil is prepared, needed is a surfactant suitable for emulsifying the silicone oil and an aqueous component such as water. As such a surfactant, a polyether-modified polyorganosiloxane etc. are known.

It is also known that, according to formulation, a polyorganosiloxane co-modified with a polysiloxane chain or a long-chain alkyl group on the side chain is used as the surfactant to yield a stably emulsified product (Patent Document 1).

The stability of the emulsified product generally has a relationship with interfacial tension, and required is a surfactant that can more reduce the interfacial tension. There have been many developments for demanding a more excellent surfactant, but there has been room for improvement on a shape of a siloxane chain for modification on the side chain. As for the shape of the siloxane chain for modification on the side chain, a linear shape described in Patent Document 2 and a dendritic shape described in Patent Document 3 have been investigated, but a siloxane with cyclic structure has not been investigated.

### CITATION LIST

### PATENT LITERATURE

Patent Document 1: JP 2001-39819 A
Patent Document 2: WO 2011/049248
Patent Document 3: JP 2002-179797 A

### SUMMARY OF INVENTION

### TECHNICAL PROBLEM

The present invention has been made in view of the above circumstances. An object of the present invention is to provide a surfactant having a high effect of reducing interfacial tension.

### SOLUTION TO PROBLEM

To achieve the above object, the present invention provides a surfactant represented by the following general formula (1), 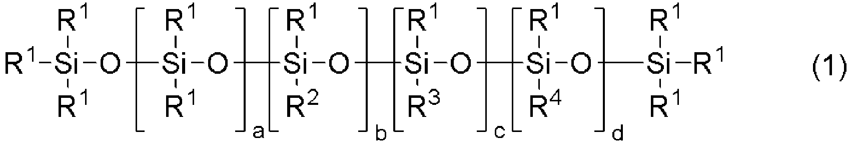 wherein R¹ independently represents a group selected from an alkyl group having 1 to 4 carbon atoms and an aryl group having 6 to 10 carbon atoms, R² independently represents a group represented by the following general formula (2), R³ independently represents an alkyl group having 6 to 20 carbon atoms, R⁴ independently represents a group having a cyclic siloxane structure represented by the following general formula (3), a = 10 to 60, b = 1 to 30, c = 0 to 30, d = 1 to 30, provided that "a", "b", "c", and "d" satisfy a range of a + b + c + d = 20 to 100, and a bonding order of siloxane units in brackets with "a" to "d" may be block or random, 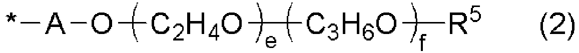 wherein A represents an alkylene group having 2 to 10 carbon atoms, R⁵ represents a hydrogen atom or an alkyl group having 1 to 10 carbon atoms, "e" represents an integer satisfying e = 0 to 100, "f" represents an integer satisfying f = 0 to 100, provided that e + f represents an integer of 2 to 200, a bonding order of alkylene oxide units in brackets with "e" and "f" may be block or random, and "*" represents an attachment bonded to a silicon atom, 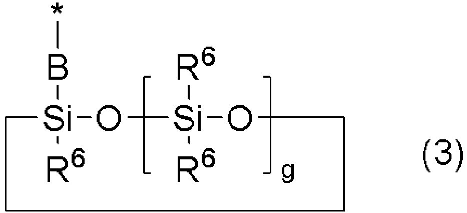 wherein B represents an alkylene group having 2 to 10 carbon atoms, R⁶ independently represents a hydrogen atom or a group selected from an alkyl group having 1 to 4 carbon atoms and an aryl group having 6 to 22 carbon atoms, "g" represents an integer satisfying g = 2 to 5, and "*" represents an attachment bonded to a silicon atom.

Such a surfactant can sufficiently reduce interfacial tension.

In an aspect of the present invention, the surfactant is preferably a cyclic siloxane wherein R⁶ represents a methyl group and g = 3 in the general formula (3).

Such a surfactant is preferable from the viewpoint of steric hindrance and easiness of forming the cyclic structure.

In addition, the present invention provides a cosmetic including the above surfactant.

Such a cosmetic has high stability with time.

In an aspect of the present invention, the cosmetic preferably has an emulsion form.

For the cosmetic of the present invention, various forms such as water-in-oil emulsion, oil-in-water emulsion, non-aqueous emulsion, and multi-emulsion such as W/O/W and O/W/O can be selected.

### ADVANTAGEOUS EFFECTS OF INVENTION

Use of the surfactant of the present invention can reduce the interfacial tension compared with a surfactant with similar structure. When the surfactant is blended in a cosmetic, a proportion of a hydrophobic moiety and a hydrophilic moiety needs to be appropriately set depending on a form of the cosmetic, and the structure of the surfactant is limited by this proportion. In the limitation, it has been found that the hydrophobic moiety is changed from a linear silicone chain to a cyclic silicone chain to more reduce the interfacial tension. In addition, blending the surfactant of the present invention can provide a cosmetic having high stability with time. The cosmetic containing the surfactant of the present invention has light extensibility without oiliness and tackiness, plain feel of use with wetness and freshness, extremely long makeup durability, and extremely excellent stability without change with temperature and time.

### DESCRIPTION OF EMBODIMENTS

There has been a demand for a surfactant having a high effect of reducing interfacial tension.

The present inventors have made earnest study to achieve the above object, and consequently found that a polyether-modified polyorganosiloxane having a cyclic polysiloxane chain on a side chain has a high effect of reducing interfacial tension. This finding has led to completion of the present invention.

Specifically, the present invention is a surfactant represented by the following general formula (1), 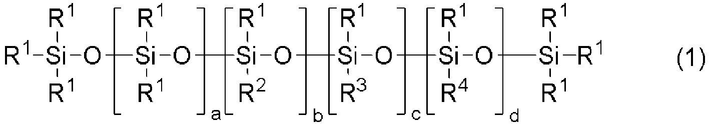 wherein R¹ independently represents a group selected from an alkyl group having 1 to 4 carbon atoms and an aryl group having 6 to 10 carbon atoms, R² independently represents a group represented by the following general formula (2), R³ independently represents an alkyl group having 6 to 20 carbon atoms, R⁴ independently represents a group having a cyclic siloxane structure represented by the following general formula (3), a = 10 to 60, b = 1 to 30, c = 0 to 30, d = 1 to 30, provided that "a", "b", "c", and "d" satisfy a range of a + b + c + d = 20 to 100, and a bonding order of siloxane units in brackets with "a" to "d" may be block or random, 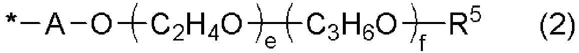 wherein A represents an alkylene group having 2 to 10 carbon atoms, R⁵ represents a hydrogen atom or an alkyl group having 1 to 10 carbon atoms, "e" represents an integer satisfying e = 0 to 100, "f" represents an integer satisfying f = 0 to 100, provided that e + f represents an integer of 2 to 200, a bonding order of alkylene oxide units in brackets with "e" and "f" may be block or random, and "*" represents an attachment bonded to a silicon atom, 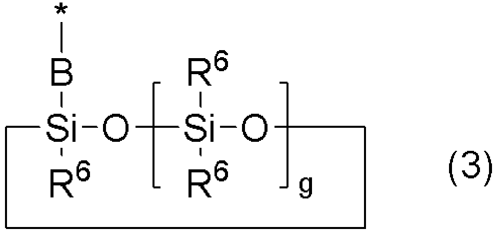 wherein B represents an alkylene group having 2 to 10 carbon atoms, R⁶ independently represents a hydrogen atom or a group selected from an alkyl group having 1 to 4 carbon atoms and an aryl group having 6 to 22 carbon atoms, "g" represents an integer satisfying g = 2 to 5, and "*" represents an attachment bonded to a silicon atom.

Hereinafter, the present invention will be described in detail.

The surfactant of the present invention is represented by the following general formula (1).

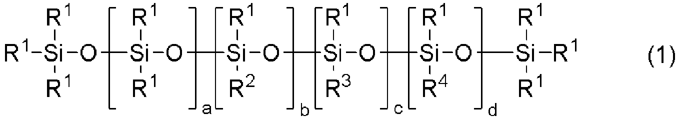

In the formula, R¹ independently represents a group selected from an alkyl group having 1 to 4 carbon atoms and an aryl group having 6 to 10 carbon atoms, R² independently represents a group represented by the following general formula (2), R³ independently represents an alkyl group having 6 to 20 carbon atoms, R⁴ independently represents a group having a cyclic siloxane structure represented by the following general formula (3), a = 10 to 60, b = 1 to 30, c = 0 to 30, d = 1 to 30, provided that "a", "b", "c", and "d" satisfy a range of a + b + c + d = 20 to 100, and a bonding order of siloxane units in brackets with "a" to "d" may be block or random,

In the general formula (1), R¹ independently represents a group selected from an alkyl group having 1 to 4 carbon atoms and an aryl group having 6 to 10 carbon atoms. From the viewpoint of easiness of orientation to the interface, R¹ preferably represents a methyl group or an ethyl group, and more preferably a methyl group.

In the general formula (1), R² independently represents a group represented by the following general formula (2).

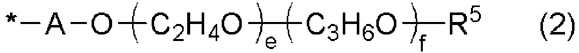

In the formula, A represents an alkylene group having 2 to 10 carbon atoms, R⁵ represents a hydrogen atom or an alkyl group having 1 to 10 carbon atoms, "e" represents an integer satisfying e = 0 to 100, "f" represents an integer satisfying f = 0 to 100, provided that e + f represents an integer of 2 to 200, a bonding order of alkylene oxide units in brackets with "e" and "f" may be block or random, and "*" represents an attachment bonded to a silicon atom.

In the general formula (2), A represents an alkylene group having 2 to 10 carbon atoms. From the viewpoint of increase in an effect by the hydrophilic group, A preferably represents an alkylene group having 2 to 4 carbon atoms, and more preferably a propylene group.

In the general formula (2), R⁵ represents a hydrogen atom or an alkyl group having 1 to 10 carbon atoms. From the viewpoint of hydrophilicity, R⁵ preferably represents a hydrogen atom or an alkyl group having 1 to 4 carbon atoms, and more preferably a hydrogen atom or a methyl group.

In the general formula (2), "e" and "f" respectively represent an integer satisfying e = 0 to 100 and an integer satisfying f = 0 to 100, and e + f represents an integer of 2 to 200. From the viewpoint of compatibility with silicone, e + f preferably represents 2 to 100, and more preferably 2 to 50.

In the general formula (2), a bonding order of alkylene oxide units in brackets with "e" and "f" may be block or random. "*" represents an attachment (free radical) bonded to a silicon atom.

In the general formula (1), R³ independently represents an alkyl group having 6 to 20 carbon atoms. From the viewpoints of hydrophobicity and steric hindrance, R³ preferably represents an alkyl group having 6 to 16 carbon atoms, and more preferably an alkyl group having 6 to 12 carbon atoms.

In the general formula (1), R⁴ independently represents a group having a cyclic siloxane structure represented by the following general formula (3).

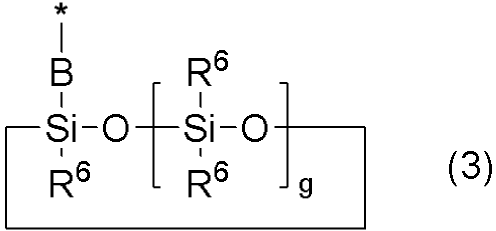

In the formula, B represents an alkylene group having 2 to 10 carbon atoms, R⁶ independently represents a hydrogen atom or a group selected from an alkyl group having 1 to 4 carbon atoms and an aryl group having 6 to 22 carbon atoms, "g" represents an integer satisfying g = 2 to 5, and "*" represents an attachment bonded to a silicon atom.

In the general formula (3), B represents an alkylene group having 2 to 10 carbon atoms. From the viewpoint of easiness of forming a bond to the main chain, B preferably represents an alkylene group having 2 to 4 carbon atoms, and more preferably an ethylene group.

In the general formula (3), R⁶ independently represents a hydrogen atom or a group selected from an alkyl group having 1 to 4 carbon atoms and an aryl group having 6 to 22 carbon atoms. From the viewpoint of steric hindrance, R⁶ preferably represents a methyl group or an ethyl group, and more preferably a methyl group.

In the general formula (3), "g" represents an integer satisfying g = 2 to 5. From the viewpoint of easiness of forming the cyclic structure, "g" preferably represents 2 or 3, and more preferably 3.

In the general formula (3), "*" represents an attachment bonded to a silicon atom.

In the general formula (1), a = 10 to 60, b = 1 to 30, c = 0 to 30, d = 1 to 30, provided that "a", "b", "c", and "d" satisfy a range of a + b + c + d = 20 to 100. From the viewpoint of easiness of handling in cosmetic preparation, "a" preferably represents 15 to 55, and more preferably 15 to 50. "b" preferably represents 1 to 20, and more preferably 1 to 10. "c" preferably represents 0 to 25, and more preferably 0 to 20. "d" preferably represents 1 to 20, and more preferably 1 to 10.

A bonding order of siloxane units in brackets with "a" to "d" may be block or random.

The surfactant represented by the general formula (1) can be produced by the following method, for example. The method for producing the surfactant described in the present invention is not limited to the following method. The surfactant can be produced by an addition reaction of an organohydrogenpolysiloxane represented by the following general formula (4), a polyoxyalkylene compound represented by the following general formula (5), and a cyclic siloxane compound represented by the following general formula (6).

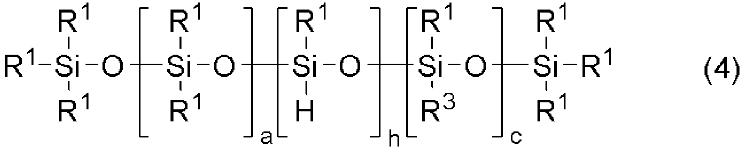

In the general formula (4), R¹, R³, "a", and "c" are same as in the general formula (1).

In the general formula (4), "h" represents a number satisfying h = b + d of "b" and "d" in the general formula (1), and a + c + h satisfies a range of 20 to 100. A bonding order of siloxane units in brackets with "a", "h", and "c" may be block or random.

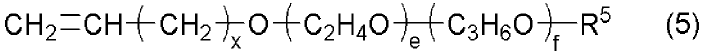

In the general formula (5), R⁵, "e", and "f" are same as in the general formula (2).

In the general formula (5), "x" represents an integer of 0 to 8. From the viewpoint of reactivity, "x" preferably represents 0 to 2, and more preferably x = 1.

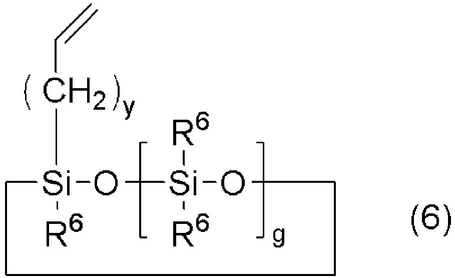

In the general formula (6), R⁶ and "g" are same as in the general formula (3).

In the general formula (6), "y" represents an integer of 0 to 8. From the viewpoint of easiness of introduction into the siloxane, "y" preferably represents 0 to 2, and more preferably y = 0.

The addition reaction is preferably performed in the presence of a platinum catalyst or a rhodium catalyst. Specifically, catalysts such as chloroplatinic acid, an alcohol-modified chloroplatinic acid, and a chloroplatinic acid-vinylsiloxane complex are suitably used. Although an amount of the catalyst used may be a catalytic amount, the amount is preferably 50 ppm or less, and particularly preferably 20 ppm or less in terms of a platinum or rhodium amount from the viewpoint of appearance of the product.

The addition reaction may be performed in an organic solvent as necessary. Examples of the organic solvent include: aliphatic alcohols such as methanol, ethanol, 2-propanol, and butanol; aromatic hydrocarbons such as toluene and xylene; aliphatic or alicyclic hydrocarbons such as n-pentane, n-hexane, and cyclohexane; and halogenated hydrocarbons such as dichloromethane, chloroform, and carbon tetrachloride. Among these, 2-propanol (isopropyl alcohol) and toluene are preferably from the viewpoint of compatibility with the raw materials.

The addition reaction condition is not particularly limited, and the reaction is preferably performed at 70 to 110°C for 1 to 10 hours. An order of addition of the polyoxyalkylene compound represented by the general formula (5) and the cyclic polysiloxane compound represented by the general formula (6) is not particularly limited. It is acceptable that addition reaction of one compound is completed, and then addition reaction of the other compound is performed. Alternatively, both the compounds may be simultaneously added.

The surfactant produced as above has a number-average molecular weight (Mn) in terms of polystyrene by GPC of preferably 2,000 to 200,000, more preferably 4,000 to 100,000, and further preferably 5,000 to 50,000. The Mn within the above molecular weight range, the effect as the surfactant is high. In the present invention, the number-average molecular weight refers to a value by gel permeation chromatography (GPC) analysis under the following condition with polystyrene as a standard substance.

### Measurement Condition

Developing solvent: Tetrahydrofuran (THF)
Flow rate: 0.6 mL/min
Detector: Differential refractive index detector (RI)
Column: TSK Guardcolumn SuperH-H
   TSKgel SuperHM-N (6.0 mm I.D. × 15 cm × 1)
   TSKgel SuperH2500 (6.0 mm I.D. × 15 cm × 1) Device: HLC 8320 GPC
   (the all are produced by Tosoh Corporation)
Column temperature: 40°C
Sample injection amount: 50 µL (THF solution at a concentration of 0.3 mass%)

The surfactant of the present invention has a group having the cyclic siloxane structure represented by the general formula (3) to more reduce the interfacial tension. It is considered that the difference in the steric structure yields more stable orientation to the water-silicone oil interface to lead to reduction of the interfacial tension. The interfacial tension can be measured by standing still a container containing water and an oil agent such as polydimethylsiloxane with a Wilhelmy method.

The surfactant of the present invention can be used for various applications, and is particularly suitable for a raw material of all cosmetics externally used for skin or hair. Specifically, the present invention provides a cosmetic including the above surfactant.

A blending amount of the surfactant of the present invention in the cosmetic is preferably 0.1 to 50 mass%, and more preferably 1 to 30 mass% in an entirety of the cosmetic. With this range, the surfactant can be stably blended in the cosmetic.

In the cosmetic of the present invention, components that can be commonly blended in a cosmetic may be appropriately blended in addition to the above surfactant. As these components, (A) an oily component, (B) an ultraviolet-ray absorbing component, (C) water, (D) a surfactant other than the surfactant of the present invention, (E) powder, (F) a compound having an alcoholic hydroxy group in a molecular structure, (G) a water-soluble or water-swellable polymer, (H) a composition containing a crosslinked organopolysiloxane polymer having no hydrophilic group and an oil agent, (I) a composition containing a crosslinked organopolysiloxane polymer having a hydrophilic group and an oil agent, (J) a silicone resin, and/or (K) a silicone wax may be contained. Hereinafter, each component will be described. Hereinafter, a compound name may be described as International Nomenclature of Cosmetic Ingredients (INCI).

### (A) Oily Component

As noted above, the cosmetic of the present invention may contain one or two or more oily components. As the oily component, any of solid, semisolid, and liquid oily components used in a common cosmetic may be used. In the present description, an oily component being liquid at 25°C refers to "oil agent".

Examples of such an oily component include one or two or more of a silicone oil, a hydrocarbon oil, a higher fatty acid, a higher alcohol, a polar oil such as an ester oil and natural animal or vegetable oil, a semisynthetic oil, and/or a fluorine oil, and a hydrocarbon oil and a silicone oil are preferable. Even when the oily component is a silicone oil, the surfactant contained in the cosmetic of the present invention has compatibility with both the components, and can provide the cosmetic having good feel of use and excellent usability and stability.

Examples of the silicone oil include: linear or branched organopolysiloxanes having low viscosity to high viscosity such as Dimethicone (INCI), Caprylyl Methicone (INCI), Phenyl Trimethicone (INCI), Hexyl Dimethicone (INCI), Hydrogen Dimethicone (INCI), and Diphenyl Dimethicone (INCI); cyclic organopolysiloxanes such as Cyclotetrasiloxane (INCI), Cyclopentasiloxane (INCI), Cyclohexasiloxane (INCI), tetrahydrotetramethylcyclotetrasiloxane, and tetramethyltetraphenylcyclotetrasiloxane; branched organopolysiloxanes such as tristrimethylsiloxymethylsilane and tetrakistrimethylsiloxysilane; amino-modified organopolysiloxanes; silicone rubbers such as a highly polymerized gummy dimethylpolysiloxane, gummy amino-modified organopolysiloxane, and gummy dimethylsiloxanemethylphenylsiloxane copolymer; cyclic siloxane solutions of a silicone gam or rubber; trimethylsiloxysilicic acid or a cyclic organopolysiloxane solution of trimethylsiloxysilicic acid; higher-alkoxy-modified organopolysiloxane such as Stearoxy Dimethicone (INCI); higher-fatty-acid-modified organopolysiloxanes; alkyl-modified organopolysiloxanes; long-chain-alkyl-modified organopolysiloxanes; fluorinemodified organopolysiloxanes; and a silicone resin and a dissolved product of a silicone resin.

Examples of the hydrocarbon oil include linear or branched, or volatile hydrocarbon oils. Examples thereof include Ozokerite (INCI), Olefin Oligomer (INCI), Isododecane (INCI), Hydrogenated Polyisobutene (INCI), Squalane (INCI), Squalene (INCI), Ceresin (INCI), Paraffin (INCI), Polyethylene (INCI), polyethylenepolypropylene wax, (ethylene/propylene/styrene) copolymer, (butylene/propylene/styrene) copolymer, Pristane (INCI), polyisobutylene, Microcrystalline Wax (INCI), and Petrolatum (INCI).

Examples of the higher fatty acid include Lauric Acid (INCI), Myristic Acid (INCI), Palmitic Acid (INCI), Stearic Acid (INCI), Behenic Acid (INCI), Undecylenic Acid (INCI), Oleic Acid (INCI), Linoleic Acid (INCI), Linolenic Acid (INCI), Arachidonic Acid (INCI), Eicosapentaenoic Acid (EPA) (INCI), Docosahexaenoic Acid (DHA) (INCI), Isostearic Acid (INCI), and Hydroxystearic Acid (INCI). Examples of the higher alcohol include Lauryl Alcohol (INCI), Myristyl Alcohol (INCI), Cetanol (INCI), Stearyl Alcohol (INCI), Behenyl Alcohol (INCI), Oleyl Alcohol (INCI), Isostearyl Alcohol (INCI), Octyldodecanol (INCI), Cetearyl Alcohol (INCI), Decyltetradecanol (INCI), Cholesterol (INCI), Phytosterols (INCI), Batyl Alcohol (INCI), and Oleyl Glyceryl Ether (INCI).

Examples of the ester oil include Diisobutyl Adipate (INCI), Diethylhexyl Adipate (INCI), Heptylundecyl Adipate (INCI), n-alkylene (20 to 30) glycol monoisostearate, Isocetyl Isostearate (INCI), Trimethylolpropane Triisostearate (INCI), ethylene glycol di-2-ethylhexanoate, Cetyl Ethylhexanoate (INCI), Trimethylolpropane Triethylhexanoate (INCI), Pentaerythrityl Tetraethylhexanoate (INCI), Cetyl Ethylhexanoate (INCI), Octyldodecyl Myristate (INCI), Oleyl Oleate (INCI), Octyldodecyl Oleate (INCI), Decyl Oleate (INCI), Neopentyl Glycol Diethylhexanoate (INCI), Neopentyl Glycol Dicaprate (INCI), Triethyl Citrate (INCI), Diethylhexyl Succinate (INCI), Amyl Acetate (INCI), Ethyl Acetate (INCI), Butyl Acetate (INCI), Isocetyl Stearate (INCI), Butyl Stearate (INCI), Diisopropyl Sebacate (INCI), Diethylhexyl Sebacate (INCI), Cetyl Lactate (INCI), Myristyl Lactate (INCI), Isononyl Isononanoate (INCI), Isotridecyl Isononanoate (INCI), Isopropyl Palmitate (INCI), Ethylhexyl Palmitate (INCI), Hexyldecyl Palmitate (INCI), Cholesteryl Hydroxystearate (INCI), Isopropyl Myristate (INCI), Octyldodecyl Myristate (INCI), 2-hexyldecyl myristate, Myristyl Myristate (INCI), hexyldecyl dimethyloctanoate, Ethylhexyl Laurate (INCI), Hexyl Laurate (INCI), Phytosteryl/Octyldodecyl Lauroyl Glutamate (INCI), Isopropyl Lauroyl Sarcosinate (INCI), and Diisostearyl Malate (INCI). Examples of glyceride oil include Glyceryl Acetate (INCI), Triethylhexanoin (INCI), Glyceryl Isostearate (INCI), Triisopalmitin (INCI), Glyceryl Stearate (INCI), Glyceryl Diisostearate (INCI), Trimyristin (INCI), and Isostearic/Myristic Glycerides (INCI).

Examples of the natural animal or vegetable oils and the semisynthetic oils include Persea Gratissima (Avocado) Oil (INCI), Linum Usitatissimum (Linseed) Seed Oil (INCI), Prunus Amygdalus Dulcis (Sweet Almond) Oil (INCI), insect wax, perilla oil, olive oil, kapok wax, torreya oil, Copernicia Cerifera (Carnauba) Wax (INCI), Shark Liver Oil (INCI), Cod Liver Oil (INCI), Euphorbia Cerifera (Candelilla) Wax (INCI), beef tallow, cattle leg fat, cattle bone fat, hardened beef tallow, prunus armeniaca (apricot) kernel oil, whale fat, Hydrogenated Palm Oil (INCI), Triticum Vulgare (Wheat) Germ Oil (INCI), Sesamum Indicum (Sesame) Seed Oil (INCI), Oryza Sativa (Rice) Germ Oil (INCI), Oryza Sativa (Rice) Bran Oil (INCI), Saccharum Officinarum (Sugarcane) Wax (INCI), Camellia Kissi Seed Oil (INCI), Carthamus Tinctorius (Safflower) Seed Oil (INCI), Butyrospermum Parkii (Shea Butter) (INCI), Cocos Nucifera (Coconut) Oil (INCI), cinnamon oil, Simmondsia Chinensis (Jojoba) Seed Wax (INCI), Shellac Wax (INCI), Turtle Oil (INCI), Glycine Soja (Soybean) Oil (INCI), Camelia Sinensis Seed Oil (INCI), Camellia Japonica Seed Oil (INCI), Oenothera Biennis (Evening Primrose) Oil (INCI), Zea Mays (Corn) Oil (INCI), Lard (INCI), rapeseed oil, tung oil, Oryza Sativa (Rice) Bran Wax (INCI), Horse Fat (INCI), persic oil, Elaeis Guineensis (Palm) Oil (INCI), Elaeis Guineensis (Palm) Kernel Oil (INCI), Ricinus Communis (Castor) Seed Oil (INCI), Hydrogenated Castor Oil (INCI), castor oil fatty acid methyl ester, sunflower oil, Vitis Vinifera (Grape) Seed Oil (INCI), Myrica cerifera wax, Jojoba Oil (INCI), Hydrogenated Jojoba Oil (INCI), Macadamia Integrifolia Seed Oil (INCI), Beeswax (INCI), Mink Oil (INCI), Limnanthes Alba (Meadowfoam) Seed Oil (INCI), Gossypium (Cotton) Seed Oil (INCI), cotton wax, Rhus Succedanea Fruit Wax (INCI), Montan Wax (INCI), Hydrogenated Coconut Oil (INCI), coconut oil fatty acid triglyceride, Arachis Hypogaea (Peanut) Oil (INCI), Lanolin (INCI), Lanolin Oil (INCI), Hydrogenated Lanolin (INCI), Lanolin Alcohol (INCI), Lanolin Wax (INCI), Acetylated Lanolin (INCI), Acetylated Lanolin Alcohol (INCI), Isopropyl Lanolate (INCI), POE lanolin alcohol ether, POE lanolin alcohol acetate, lanolin fatty acid polyethylene glycol, POE hydrogenated lanolin alcohol ether, and Egg Oil (INCI). Note that "POE" means polyoxyethylene.

Examples of the fluorine oil agent include perfluoro polyether, Perfluorodecaline (INCI), and perfluorooctane.

Although depending on the agent type of the cosmetic, the content of the oily component (A) contained in the cosmetic of the present invention is preferably 1 to 98 mass%, and more preferably 1 to 50 mass% in the entirety of the cosmetic.

### (B) Ultraviolet-Ray Absorbing Component

The cosmetic of the present invention may further contain one or two or more ultraviolet-ray absorbing components. Accordingly, the cosmetic of the present invention becomes a cosmetic that has good feel of use, excellent usability and retainability, and that can absorb ultraviolet ray. The ultraviolet-ray absorbing component encompasses an ultraviolet-ray absorbent and an ultraviolet-ray scattering agent. Examples of the ultraviolet-ray absorbent include benzoic-acid-based ultraviolet-ray absorbents such as PABA (INCI), anthranilic acid-based ultraviolet-ray absorbents such as Methyl Anthranilate (INCI), salicylic-acid-based ultraviolet-ray absorbents such as Methyl Salicylate (INCI), cinnamic-acid-based ultraviolet-ray absorbents such as Ethylhexyl Methoxycinnamate (INCI), benzophenone-based ultraviolet-ray absorbents such as Benzophenone-1 (INCI), urocanic-acid-based ultraviolet-ray absorbents such as ethyl urocanate, and dibenzoylmethane-based ultraviolet-ray absorbents such as Butyl Methoxydibenzoylmethane (INCI). The aforementioned silicone derivative having an ultraviolet-ray absorbing functional group may also be used. Examples of the ultraviolet-ray scattering agent include powder that absorbs and scatters ultraviolet ray such as titanium oxide fine particles, iron-containing titanium oxide fine particle, zinc oxide fine particles, cerium oxide fine particles, and a composite thereof. Among these, the cinnamic-acid-based ultraviolet-ray absorbent, the dibenzoylmethane-based ultraviolet-ray absorbent, titanium oxide, and zinc oxide are preferable. A blending amount of the ultraviolet-ray absorbing component is preferably 1 to 80 mass%, and more preferably 20 to 60 mass% in the entirety of the cosmetic. Within this range, the effect of absorbing ultraviolet ray can be stably obtained.

### (C) Water

In the cosmetic of the present invention, water may be blended according to the purpose. Blending water in the cosmetic of the present invention according to the use purpose yields a cosmetic having more excellent usability. The blending amount of water is preferably within a range of 95 mass% or less in the entirety of the cosmetic.

### (D) Surfactant other than that of the Present Invention

The cosmetic of the present invention may further contain one or two or more surfactants. Blending another surfactant in the cosmetic of the present invention according to the use purpose yields a cosmetic having more excellent usability. Examples of the other surfactant include anionic, cationic, nonionic, and amphoteric surfactants. The surfactant contained in the cosmetic of the present invention is not particularly limited, and any surfactant used in a common cosmetic may be used.

Examples of the anionic surfactant include fatty acid soap such as Sodium Stearate (INCI) and TEA-Palmitate (INCI), an alkyl ether carboxylic acid and a salt thereof, a condensate salt of an amino acid and a fatty acid, an alkanesulfonate salt, an alkenesulfonate salt, a sulfonate salt of a fatty acid ester, a sulfonate salt of a fatty acid amide, a formalincondensed sulfonate salt, an alkylsulfate ester salt, a secondary higher alcohol sulfate ester salt, alkyl and allyl ether sulfate ester salts, a sulfate ester salt of a fatty acid ester, a sulfate ester salt of a fatty acid alkylolamide, sulfate ester salts such as Sulfated Castor Oil (INCI), an alkylphosphate salt, an ether phosphate salt, an alkyl allyl ether phosphate salt, an amidophosphate salt, an N-acyllactate salt, an N-acylsarcosinate salt, and an N-acylamino acid-based activator. Examples of the cationic surfactant include amine salts such as an alkylamine salt, a polyamine, an aminoalcohol fatty acid derivative, an alkyl quaternary ammonium salt, an aromatic quaternary ammonium salt, a pyridinium salt, and an imidazolium salt.

Examples of the nonionic surfactant include a sorbitan fatty acid ester, a glycerol fatty acid ester, a polyglycerol fatty acid ester, a propylene glycol fatty acid ester, a polyethylene glycol fatty acid ester, a sucrose fatty acid ester, a methylglucoside fatty acid ester, an alkylpolyglucoside, a polyoxyethylene alkyl ether, a polyoxypropylene alkyl ether, a polyoxyethylene alkyl phenyl ether, a polyoxyethylene fatty acid ester, a polyoxyethylene sorbitan fatty acid ester, a polyoxyethylene sorbitol fatty acid ester, a polyoxyethylene glycerol fatty acid ester, a polyoxyethylene propylene glycol fatty acid ester, polyoxyethylene castor oil, polyoxyethylene hardened castor oil, polyoxyethylene phytostanol ether, polyoxyethylene phytosterol ether, polyoxyethylene cholestenol ether, polyoxyethylene cholesterol ether, a linear or branched polyoxyalkylene-modified organopolysiloxane, a linear or branched polyoxyalkylene-alkyl-co-modified organopolysiloxane, a linear or branched polyglycerol-modified organopolysiloxane, a linear or branched polyglycerol-alkyl-co-modified organopolysiloxane, an alkanolamide, a saccharide ether, and a saccharide amide.

Examples of the amphoteric surfactant include a betaine, an aminocarboxylate salt, an imidazoline derivative, and an amidoamine-based surfactant.

As the surfactant, the polyether-modified polyorganosiloxane of the present invention may be used singly, or the other surfactant exemplified above may also be used in combination. A blending amount of the surfactant is preferably 0.1 to 50 mass%, and particularly preferably a range of 1 to 30 mass% in the entirety of the cosmetic. Within this range, the surfactant can be stably blended.

### (E) Powder

The cosmetic of the present invention may further contain one or two or more powders. As the powder, any powder may be used regardless of the shape (such as spherical, needle, and platelet shapes), the particle diameter (such as fume, fine particles, and pigmentlevel), and the particle structure (such as porous and nonporous structure) as long as the powder is used for a common cosmetic. Examples of the powder include inorganic powder, organic powder, surfactant metal salt powder, and coloring agents such as a colored pigment, a pearl pigment, a tar coloring matter, a metal powder pigment, a natural coloring matter, and a dye.

Examples of the inorganic powder include Titanium Dioxide (INCI), Zirconium Dioxide (INCI), Zinc Oxide (INCI), Cerium Oxide (INCI), magnesium oxide, barium sulfate, calcium sulfate, magnesium sulfate, calcium carbonate, magnesium carbonate, Talc (INCI), Mica (INCI), Kaolin (INCI), sericite, synthetic mica, phlogopite, lepidolite, biotite, lithia mica, Silica (INCI), Aluminum Silicate (INCI), Magnesium Silicate (INCI), Magnesium Aluminum Silicate (INCI), Calcium Silicate (INCI), barium silicate, strontium silicate, a metal tungstate salt, Hydroxyapatite (INCI), vermiculite, heidilite, Bentonite (INCI), Montmorillonite (INCI), Hectorite (INCI), Zeolite (INCI), calcium phosphate dibasic, Alumina (INCI), Aluminum Hydroxide (INCI), Boron Nitride (INCI), and Silica Silylate (INCI).

Examples of the organic powder include polyamide powder, polyacrylic-acid-acrylate ester powder, polyester powder, polyethylene powder, polypropylene powder, polystyrene powder, polyurethane, benzoguanamine powder, polymethylbenzoguanamine powder, tetrafluoroethylene powder, polymethyl methacrylate powder, Cellulose (INCI), silk powder, nylon powder, Nylon-12 (INCI), Nylon-6 (INCI), crosslinked spherical dimethylpolysiloxane fine powder having structure of crosslinked dimethylpolysiloxane, crosslinked spherical polymethylsilsesquioxane fine powder, fine powder in which a surface of crosslinked spherical organopolysiloxane rubber is coated with polymethylsilsesquioxane particles, hydrophobized silica, styrene-acrylic acid copolymer, divinylbenzene-styrene copolymer, vinyl resin, urea resin, phenol resin, fluorine resin, silicon resin, acrylic resin, melamine resin, epoxy resin, polycarbonate resin, fine crystal fiber powder, starch powder, fatty acid starch derivative powder, and Lauroyl Lysin (INCI).

Examples of the surfactant metal salt powder (meal soap) include Zinc Undecylenate (INCI), Aluminum Isostearate (INCI), Zinc Stearate (INCI), Aluminum Stearate (INCI), Calcium Stearate (INCI), Magnesium Stearate (INCI), Zinc Myristate (INCI), Magnesium Myristate (INCI), Sodium Zinc Cetyl Phosphate (INCI), calcium cetyl phosphate, Zinc Palmitate (INCI), aluminum palmitate, and Zinc Laurate (INCI).

Specific examples of the colored pigment include: inorganic red pigments such as iron oxide, iron hydroxide, and iron titanate; inorganic brownish pigments such as γ-iron oxide; inorganic yellowish pigment such as yellow iron oxide and ocher; inorganic black pigments such as black iron oxide and Carbon Black (INCI); inorganic violet pigments such as Manganese Violet (INCI) and cobalt violet; inorganic green pigments such as Chromium Hydroxide Green (INCI), Chromium Oxide Greens (INCI), cobalt oxide, and Cobalt Titanium Oxide (INCI); inorganic blueish pigments such as Prussian blue and ultramarine blue; a lake of tar coloring matters, a lake of natural coloring matters, and synthetic resin powder of a composite of these powders.

Specific examples of the pearl pigment include titanium-oxide-coated mica, bismuth oxychloride, titanium-oxide-coated bismuth oxychloride, titanium-oxide-coated talc, fish scales, and titanium-oxide-coated colored mica. Examples of the metal powder pigment include aluminum powder, copper powder, and stainless steel powder.

Examples of the tar coloring matter include Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207. Examples of the natural coloring matter include powder selected from carminic acid, Laccaic Acid (INCI), carthamin, brazilin, crocin, etc.

Among these powders, preferable in the present invention are crosslinked spherical dimethylpolysiloxane fine powder having crosslinked structure with dimethylpolysiloxane, crosslinked spherical polymethylsilsesquioxane fine powder, fine powder in which a surface of crosslinked spherical polysiloxane rubber is coated with polymethylsilsesquioxane, fine powder in which a surface of crosslinked spherical diphenylpolysiloxane rubber is coated with polymethylsilsesquioxane particles, and hydrophobized silica. Powder and a coloring agent having a fluorine group are also used. Examples of the commercially available product include KMP-590, KSP-100, KSP-101, KSP-102, KSP-105, and KSP-300 (all are produced by Shin-Etsu Chemical Co., Ltd.).

According to the purpose, these powders may be subjected to composite formation of the powder or treated with a general oil agent, silicone oil, a fluorine compound, a surfactant, etc. for use. For example, the powder may or may not be subjected to surface treatment in advance with fluorine-compound treatment, silicone-resin treatment, pendant treatment, silane-coupling-agent treatment, titanium-coupling-agent treatment, oil-agent treatment, N-acylated-lysine treatment, polyacrylic-acid treatment, metal-soap treatment, amino-acid treatment, inorganic-compound treatment, plasma treatment, mechanochemical treatment, etc. One or two or more of the treatment may be used as necessary. A blending amount of these powders is preferably within a range of 99 mass% or less in the entirety of the cosmetic. Particularly, the blending amount in a case of a powder cosmetic is preferably within a range of 80 to 99 mass% in the entirety of the cosmetic.

### (F) Compound having Alcoholic Hydroxy Group in Molecule Structure

The cosmetic of the present invention may further contain one or more compounds having an alcoholic hydroxy group in the molecule structure. Examples of such a compound include: lower alcohols such as Alcohol (INCI) and Isopropyl Alcohol (INCI); saccharide alcohols such as Sorbitol (INCI) and Maltose (INCI); sterols such as Cholesterol (INCI), Beta-Sitosterol (INCI), Phytosterols (INCI), and Lanosterol (INCI); and polyhydric alcohols such as Butylene Glycol (INCI), Propylene Glycol (INCI), Dipropylene Glycol (INCI), and Pentylene Glycol (INCI). In typical, a water-soluble monohydric alcohol or a water-soluble polyhydric alcohol is used. A blending amount of the compound having an alcoholic hydroxy group in the molecule structure is preferably within a range of 98 mass% or less in the entirety of the cosmetic.

### (G) Water-Soluble or Water-Swellable Polymer

The cosmetic of the present invention may further contain one or two or more water-soluble or water swellable polymers (G). Examples of these water-soluble or water-swellable polymers include: vegetable polymers such as Acacia Senegal Gum (INCI), Astragalus Gummifer Gum (INCI), galactan, Ceratonia Siliqua Gum (INCI), Cyamopsis Tetragonoloba (Guar) Gum (INCI), Sterculia Urens Gum (INCI), Chondrus Crispus (Carrageenan) (INCI), Pectin (INCI), Agar (INCI), Pyrus Cydonia Seed (INCI), Oryza Sativa (Rice) Starch (INCI), Triticum Vulgare (Wheat) Starch (INCI), and Solanum Tuberosum (Potato) Starch (INCI); microbiological polymers such as Xanthan Gum (INCI), Dextran (INCI), Succinoglycan (INCI), and Pullulan (INCI); animal polymers such as Collagen (INCI), Casein (INCI), albumin, and Gelatin (INCI); starch polymers such as Sodium Carboxymethyl Starch (INCI) and Hydroxypropyl Starch (INCI); cellulose polymers such as Methylcellulose (INCI), Ethylcellulose (INCI), Hydroxypropylmethylcellulose (INCI), carboxymethylcellulose, hydroxymethylcellulose, Hydroxypropylcellulose (INCI), Nitrocellulose (INCI), Sodium Cellulose Sulfate (INCI), sodium carboxymethylcellulose, Microcrystalline Cellulose (INCI), and cellulose powder; alginic acid polymers such as Algin (INCI) and Propylene Glycol Alginate (INCI); vinyl polymers such as polyvinyl methyl ether and carboxyvinyl polymer; polyoxyethylene polymer; polyoxyethylene-polyoxypropylene copolymer; acrylic polymers such as Sodium Polyacrylate (INCI), Polyethylacrylate (INCI), Polyacrylamide (INCI), and Acrylamide/Sodium Acryloyldimethyltaurate Copolymer (INCI); other synthetic water-soluble polymers such as polyethylene imine and a cation polymer; and inorganic water-soluble polymers such as Bentonite (INCI), Magnesium Aluminum Silicate (INCI), Montmorillonite (INCI), beidellite, nontronite, saponite, Hectorite (INCI), and Silica (INCI). These water-soluble polymers include a film-forming agent such as Polyvinyl Alcohol (INCI) and PVP (INCI). A blending amount of the water-soluble or water-swellable polymer (G) is preferably within a range of 25 mass% or less in the entirety of the cosmetic.

### (H) Composition containing Crosslinked

### Organopolysiloxane Polymer having no Hydrophilic Group and Oil Agent

The cosmetic of the present invention may further contain one or two or more compositions (H) containing a crosslinked organopolysiloxane polymer having no hydrophilic group and an oil agent. This crosslinked organopolysiloxane polymer is obtained by a reaction of an alkylhydrogenpolysiloxane and a crosslinking agent having a hydrosilylation-reactive unsaturated group.

Examples of the alkylhydrogenpolysiloxane include methylhydrogenpolysiloxane having a linear or partially branched unit and methylhydrogenpolysiloxane grafted with an alkyl chain having 6 to 20 carbon atoms. A number of hydrogen atoms bonded to a silicon atom is necessarily two or more in average in the molecule.

Examples of the crosslinking agent include a compound having two or more carbon-carbon double bonds in the molecule that can be subjected to a hydrosilylation reaction, such as methylvinylpolysiloxane and α,ω-alkenyldiene.

The obtained crosslinked organopolysiloxane polymer is swelled with, for example, an oil agent having a kinematic viscosity of 0.65 mm²/sec (25°C) to 100.0 mm²/sec (25°C), such as hydrocarbon oil such as low viscous silicone, liquid paraffin, squalane, isododeceane, glyceride oil such as trioctanoin, and ester oil, at a weight of greater than or equal to the weight of the crosslinked organopolysiloxane polymer.

In the present invention, "kinematic viscosity" refers to a value measured at 25°C by using Cannon-Fenske viscosimeter described in JIS Z 8803:2011.

These compositions of the crosslinked organopolysiloxane polymer and the oil agent are available as a commercial product. Examples thereof include: KSG-15, KSG-16, KSG-18, KSG-1610, and USG-103, which are pastes with silicone oil; and USG-106, KSG-41, KSG-42, KSG-43, KSG-44, and KSG-810, which are pastes with hydrocarbon oil or triglyceride oil (all are produced by Shin-Etsu Chemical Co., Ltd.). A blending amount of the composition (H) containing the crosslinked organopolysiloxane polymer having no hydrophilic group and the oil agent is preferably 0.1 to 50 mass%, and further preferably 1 to 30 mass% relative to a total amount of the cosmetic.

### (I) Composition containing Crosslinked

### Organopolysiloxane Polymer having Hydrophilic Group and Oil Agent

The cosmetic of the present invention may further contain one or two or more compositions containing a crosslinked organopolysiloxane polymer having a hydrophilic group and an oil agent. The hydrophilic group is preferably a polyether group or a polyglycerol group. This crosslinked organopolysiloxane polymer having a polyether group and/or a polyglycerol group is obtained by a reaction of an alkylhydrogenpolysiloxane and a crosslinking agent having a hydrosilylation-reactive unsaturated group.

Examples of the alkylhydrogenpolysiloxane include methylhydrogenpolysiloxane grafted with a polyoxyethylene chain and methylhydrogenpolysiloxane grafted with a polyglycerol chain. A number of hydrogen atoms bonded to a silicon atom is necessarily two or more in average in the molecule.

Examples of the crosslinking agent include a compound having two or more carbon-carbon double bonds in the molecule that can be subjected to a hydrosilylation reaction, such as methylvinylpolysiloxane, α,ω-alkenyldiene, glycerol triallyl ether, polyoxyalkynylated glycerol triallyl ether, trimethylolpropane triallyl ether, and polyoxyalkynylated trimethylolpropane triallyl ether. The crosslinked product reacted from these compounds has one or more hydrophilic groups.

The obtained crosslinked organopolysiloxane polymer is swelled in an oil agent having a kinematic viscosity of 0.65 mm²/sec (25°C) to 100.0 mm²/sec (25°C), such as hydrocarbon oil such as low viscous silicone, liquid paraffin, squalane, isododeceane, glyceride oil such as trioctanoin, and ester oil, at a weight of greater than or equal to the weight of the crosslinked organopolysiloxane polymer.

These crosslinked organopolysiloxanes is available as a commercial product. Examples thereof include: KSG-210, KSG-240, and KSG-710, which are pastes with silicone oil; and KSG-310, KSG-320, KSG-330, KSG-340, KSG-820, KSG-830, and KSG-840, which are pastes with hydrocarbon oil or triglyceride oil (all are produced by Shin-Etsu Chemical Co., Ltd.). A blending amount of the composition (I) containing the crosslinked organopolysiloxane having a hydrophilic group and the oil agent is preferably 0.1 to 50 mass%, and further preferably 1 to 30 mass% relative to the total amount of the cosmetic.

### (J) Silicone Resin

The cosmetic of the present invention may further contain one or more silicone resins (J). The silicone resin is preferably selected from the group consisting of a silicone-mesh compound having a SiO_{4/2} unit and/or a R'SiO_{3/2} (R' represents an alkyl group), a linear acryl/silicone graft, or a block copolymer thereof. The linear acryl/silicone graft or the block copolymer may have one or more selected from a pyrrolidone moiety, a long-chain alkyl moiety, a polyoxyalkylene moiety, a fluoroalkyl moiety, and an anion moiety such as a carboxylic acid. Examples of the commercial product thereof include, but not particularly limited to, KP-541, KP-543, KP-545, KP-549, KP-550, KP-571, KP-575, and KP-581, which are dissolved in silicone oil, hydrocarbon oil, or an alcohol (all are produced by Shin-Etsu Chemical Co., Ltd.).

The silicone-mesh compound is preferably a silicone-mesh compound represented by MQ, MDQ, MT, MDT, or MDTQ. Note that M, D, T, and Q respectively represent a R₃SiO_{1/2} unit, a R²SiO_{2/2} unit, a RSiO_{3/2} unit, and a SiO_{4/2} unit. The silicone-mesh compound may have one or more selected from a pyrrolidone moiety, a long-chain alkyl moiety, a polyoxyalkylene moiety, a fluoroalkyl moiety, and an amino moiety in the molecule. Examples of the commercial product thereof include, but not particularly limited to, KF-7312J, KF-7312K, and KF-7312T (all are produced by Shin-Etsu Chemical Co., Ltd.).

The silicone resin (J) may be dissolved in lowviscous silicone oil, volatile silicone oil, and another solvent. In any cases, a blending amount of the silicone resin is preferably 0.1 to 20 mass%, and further preferably 1 to 10 mass% as the resin amount relative to the total amount of the cosmetic of the present invention.

### (K) Silicone Wax

The cosmetic of the present invention may contain a silicone wax (K) according to the purpose. This silicone wax is preferably a polylactone-modified polysiloxane in which polylactone being a ring-opening polymer of a five or more membered lactone compound is bonded. Alternatively, this silicone wax is preferably an acryl-modified polysiloxane having one or more functional groups selected from a pyrrolidone group, a long-chain alkyl group, a polyoxyalkylene group, a fluoroalkyl group, and an anion group such as a carboxylic acid in the molecule. Examples of the commercial product thereof include KP-561P and KP-562P, which are wax having a long-chain alkyl group (both are produced by Shin-Etsu Chemical Co., Ltd.).

Alternatively, this silicone wax is preferably a silicone-modified olefin wax obtained by an addition reaction between olefin wax and an organohydrogenpolysiloxane having one or more SiH bonds in one molecule. The olefin wax is obtained by copolymerizing ethylene and one or more dienes or obtained by copolymerizing ethylene, one or more olefins selected from α-olefins having 3 to 12 carbon atoms, and one or more dienes. This diene is preferably vinylnorbornene.

Whichever the silicone wax is selected, the blending amount when the silicone wax is used is preferably 0.1 to 20 wt%, and particularly preferably 1 to 10 wt% relative to the total amount of the cosmetic of the present invention.

### Other Components

In the cosmetic of the present invention, components used for a common cosmetic, for example, an oil-soluble gelling agent, an antiperspirant, a moisturizing agent, a bacteria-preventing and antiseptic agent, an antibacterial agent, salts, an oxidation inhibitor, a pH regulator, a chelating agent, a refreshing agent, an anti-inflammatory agent, skin-beautifying components (such as a whitening agent, a cell activator, a rough skin improver, a blood circulation promoter, a skin astringent, and an anti-seborrhoea agent), vitamins, amino acids, a nucleic acid, a hormone, a polymer compound for hair fixing, etc. may be added.

Examples of the oil-soluble gelling agent include one or two or more oil-soluble gelling agents selected from: metal soap such as Aluminum Stearate (INCI), Magnesium Stearate (INCI), and Zinc Myristate (INCI); amino acid derivatives such as Lauroyl Glutamic Acid (INCI); dextrin fatty acid esters such as Dextrin Palmitate (INCI), Dextrin Isostearate (INCI), and Dextrin Palmitate/Ethylhexanoate (INCI); sucrose fatty acid esters such as Sucrose Palmitate (INCI) and Sucrose Stearate (INCI); fructooligosaccharide fatty acid esters such as Fructooligosaccharide Caprylate (INCI) and Fructooligosaccharide Hexyldecanoate (INCI); benzylidene derivatives of sorbitol such as monobenzylidene sorbitol and dibenzylidene sorbitol; organic-modified clay minerals such as dimethylbenzyldodecylammonium montmorillonite clay and dimethyldioctadecylammonium montmorillonite clay; etc.

Examples of the antiperspirant include one or two or more antiperspirants selected from Aluminum Chlorohydrate (INCI), Aluminum Chloride (INCI), aluminum sesquichlorohydrate, zirconyl hydroxychloride, aluminum zirconium hydroxychloride, aluminum zirconium glycine complex, etc.

Examples of the moisturizing agent include one or two or more moisturizing agents selected from Glycerin (INCI), Sorbitol (INCI), PG (INCI), DPG (INCI), BG (INCI), Pentylene Glycol (INCI), Glucose (INCI), Xylitol (INCI), Maltitol (INCI), polyethylene glycol, Hyaluronic Acid (INCI), chondroitin sulfate, pyrrolidone carboxylate salt, polyoxymethylene methyl glucoside, polyoxypropylene methyl glucoside, etc.

Examples of the bacteria-preventing and antiseptic agent include one or two or more bacteria-preventing and antiseptic agents selected from a paraoxybenzoic acid alkyl ester, Benzoic Acid (INCI), Sodium Benzoate (INCI), Sorbic Acid (INCI), Potassium Sorbate (INCI), Phenoxyethanol (INCI), etc.

Examples of the antibacterial agent include one or two or more antibacterial agents selected from benzoic acid, salicylic acid, Phenol (INCI), sorbic acid, a paraoxybenzoic acid alkyl ester, p-Chloro-m-Cresol (INCI), Hexachlorophene (INCI), Benzalkonium Chloride (INCI), Chlorhexidine Dihydrochloride (INCI), Triclocarban (INCI), a photosensitizer, phenoxyethanol, etc.

Examples of the salts include inorganic salts, organic acid salts, amine salts, and amino acid salts. Examples of the inorganic salt include a sodium salt, a potassium salt, a magnesium salt, a calcium salt, an aluminum salt, a zirconium salt, and a zinc salt of an inorganic acid such as hydrochloric acid, sulfuric acid, carbonic acid, and nitric acid. Examples of the organic acid salt include a salt of organic acids such as Acetic Acid (INCI), Dehydroacetic Acid (INCI), Citric Acid (INCI), Malic Acid (INCI), Succinic Acid (INCI), Ascorbic Acid (INCI), and Stearic Acid (INCI). Examples of the amine salt and the amino acid salt include one or two or more salts selected from salts of amines such as Triethanolamine (INCI), and salts of amino acids such as Glutamic Acid (INCI). In addition, Hyaluronic Acid (INCI), a salt of chondroitin sulfate etc., Aluminum Zirconium Tetrachlorohydrate (INCI), etc., and a neutralized salt of acid-alkali used in cosmetic formulation may also be used.

Examples of the oxidation inhibitor include one or two or more oxidation inhibitors selected from Tocopherol (INCI), BHA (INCI), BHT (INCI), Phytic Acid (INCI), etc.

Examples of the pH regulator include one or two or more pH regulators selected from Lactic Acid (INCI), Citric Acid (INCI), Glycolic Acid (INCI), Citric Acid (INCI), Tartaric Acid (INCI), Malic Acid (INCI), Potassium Carbonate (INCI), Sodium Bicarbonate (INCI), Ammonium Bicarbonate (INCI), etc.

Examples of the chelating agent include one or two or more chelating agents selected from Alanine (INCI), Disodium EDTA (INCI), Sodium Polyphosphate (INCI), Sodium Metaphosphate (INCI), Phosphoric Acid (INCI), etc.

Examples of the refreshing agent include one or two or more refreshing agents selected from Menthol (INCI), Camphor (INCI), etc.

Examples of the anti-inflammatory agent include one or two or more anti-inflammatory agents selected from Allantoin (INCI), Glycyrrhizic Acid (INCI) and a salt thereof, Glycyrrhetinic Acid (INCI), Stearyl Glycyrrhetinate (INCI), tranexamic acid, Azulene (INCI), etc.

Examples of the skin-beautifying component include one or two or more skin-beautifying components selected from: whitening agents such as Placental Extract (INCI), Arbutin (INCI), Glutathione (INCI), and Saxifraga Sarmentosa Extract (INCI); cell activators such as Royal Jelly (INCI), a photosensitizer, a cholesterol derivative, and calf blood extract; rough skin improvers; blood circulation promoters such as nonanoic acid vanillylamide, Benzyl Nicotinate (INCI), nicotinic acid β-butoxyethyl ester, Capsaicin (INCI), Acetyl Zingerone (INCI), cantharides tincture, Ichthammol (INCI), Caffeine (INCI), Tannic Acid (INCI), Borneol (INCI), Tocopheryl Nicotinate (INCI), inositol hexanicotinate, cyclandelate, cinnarizine, tolazoline, acetylcholine, verapamil, cepharanthine, and Oryzanol (INCI); skin astringents such as Zinc Oxide (INCI) and Tannic Acid (INCI); anti-seborrhoea agents such as Sulfur (INCI) and thianthol; etc.

Examples of the vitamins include one or two or more vitamins selected from: vitamin A such as vitamin A oil, Retinol (INCI), Retinyl Acetate (INCI), and Retinyl Palmitate (INCI); vitamin B such as vitamin B2 such as Riboflavin (INCI), Riboflavin Tetrabutyrate (INCI), and Disodium Flavin Adenine Dinucleotide (INCI), vitamin B6 such as Pyridoxine HCl (INCI), pyridoxine dioctanoate, and Pyridoxine Dipalmitate (INCI), vitamin B12 and a derivative thereof, and vitamin B15 and a derivative thereof; vitamin C such as Ascorbic acid (INCI), Ascorbyl Dipalmitate (INCI), Disodium Ascorbyl Sulfate (INCI), and L-ascorbic acid dipotassium phosphate diester; vitamin D such as Ergocalciferol (INCI) and Cholecalciferol (INCI); vitamin E such as Tocopherol (INCI), Tocopheryl Acetate (INCI), Tocopheryl Nicotinate (INCI), and Tocopheryl Succinate (INCI); vitamin P such as Biotin (INCI) and Rutin (INCI); nicotinic acids such as Niacin (INCI), Benzyl Nicotinate (INCI), and Niacinamide (INCI); and pantothenic acids such as Calcium Pantothenate (INCI), Panthenol (INCI), pantothenyl ethyl ether, and acetylpantothenyl ethyl ether.

Examples of the amino acids include one or two or more amino acids selected from Glycine (INCI), Valine (INCI), Leucine (INCI), Isoleucine (INCI), Serine (INCI), Threonine (INCI), Phenylalanine (INCI), Arginine (INCI) Lysine (INCI), Aspartic Acid (INCI), Glutamic Acid (INCI), Cystine (INCI) Cysteine (INCI), Methionine (INCI), Tryptophan (INCI), etc.

Examples of the nucleic acid include one or two or more nucleic acids selected from DNA (INCI).

Examples of the hormone include one or two or more hormones selected from Estradiol (INCI), Ethynylestradiol (INCI), etc.

Examples of the polymer compound for hair fixing include amphoteric, anionic, cationic, and nonionic polymer compounds, and include one or two or more polymer compounds for hair fixing selected from polyvinylpyrrolidone-based polymer compounds such as PVP (INCI) and (VP/VA) Copolymer (INCI), acidic vinyl-ether-based polymer compounds such as PVM/MA Copolymer (INCI), acidic polyvinyl-acetate-based polymers such as VA/Crotonates Copolymer (INCI), acidic acryl-based polymer compounds such as (meth)acrylic acid / alkyl (meth)acrylate copolymer and (meth)acrylic acid / alkyl (meth)acrylate / alkyl acrylamide copolymer, and amphoteric acryl-based polymer compounds such as **N-**methacryloylethyl-N,N-dimethylammonium α-N-methylcarboxybetaine / alkyl (meth)acrylate copolymer and (octyl acrylamide / hydroxypropyl acrylate / butylaminoethyl methacrylate) copolymer. In addition, polymer compounds derived from natural products such as cellulose or a derivative thereof, keratin and collagen or a derivative thereof may also be suitably used.

In addition, a resin, a perfume, and a clathrate compound may also be blended as the other component.

Examples of the cosmetic of the present invention include: skin-care cosmetics such as milky lotion, cream, cleansing, pack, massage material, cosmetic essence, cosmetic oil, washing agent, deodorant, hand cream, lip cream, and wrinkle concealer; make-up cosmetics such as make-up foundation, concealer, white powder, liquid foundation, oily foundation, cheek rouge, eyeshadow, mascara, eye liner, eyebrow, and lipstick; hair cosmetics such as shampoo, rinse, treatment, and setting agent; antiperspirant; and ultraviolet-ray protecting cosmetics such as sunscreen oil, sunscreen milky lotion, and sunscreen cream.

As a shape of these cosmetics, various shapes such as liquid, milky lotion, cream, solid, paste, gel, powder, press, multilayer, mousse, cray, and stick, may be selected.

Further, as a form of these cosmetics, various forms such as aqueous form, oily form, water-in-oil emulsion, oil-in-water emulsion, non-aqueous emulsion, and multi-emulsion such as W/O/W or O/W/O emulsion may be selected. Among these, an emulsion form is preferable.

### EXAMPLE

Hereinafter, the present invention will be specifically described with showing Examples and Comparative Examples, but the present invention is not limited to the following Examples. Unless otherwise specifically described in the following examples, "%" in a composition represents "mass%", and a proportion represents a mass proportion.

### Example 1

Into a separable flask, an organohydrogenpolysiloxane (200 g, number of moles of hydrosilyl groups: 0.268 mol) with an average composition represented by the following general formula (7), a cyclic polysiloxane (33.0 g, 0.107 mol) represented by the following general formula (8), and a 3% ethanol solution of chloroplatinic acid (10 mg) were added, and the mixture was stirred at 90°C under a nitrogen gas flow for 1 hour. Thereafter, a polyoxyalkylene compound (80.8 g, number of moles of allyl groups: 0.170 mol) with an average composition represented by the following general formula (9), isopropyl alcohol (113.1 g), and a 3% ethanol solution of chloroplatinic acid (20 mg) were added, and the mixture was further stirred for 5 hours. After the reaction was finished, the solvent was distilled off (at 125°C, 1 kPa or less). Finally, celite was added into the obtained oily compound, the mixture was well mixed, and then filtered for removing an unreacted polyoxyalkylene compound to obtain a target compound (colorless and clear oil, yield: 241.6 g). The number-average molecular weight was Mn = 7646. This compound was a compound represented by the general formula (1), wherein a = 42, b = 3, c = 0, d = 2, e = 9, f = 0, and g = 3.

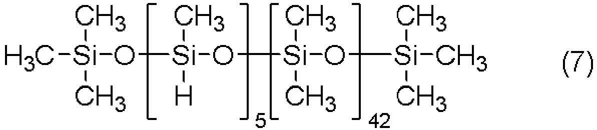

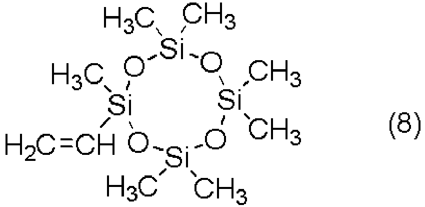

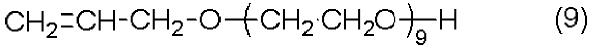

### Example 2

Into a separable flask, an organohydrogenpolysiloxane (40.0 g, number of moles of hydrosilyl groups: 0.292 mol) with an average composition represented by the following general formula (10), a cyclic polysiloxane (67.6 g, 0.219 mol) represented by the general formula (8), and a 3% ethanol solution of chloroplatinic acid (10 mg) were added, and the mixture was stirred at 90°C under a nitrogen gas flow for 1 hour. Thereafter, a polyoxyalkylene compound (36.6 g, number of moles of allyl groups: 0.077 mol) with an average composition represented by the general formula (9), isopropyl alcohol (50.4 g), and a 3% ethanol solution of chloroplatinic acid (20 mg) were added, and the mixture was further stirred for 5 hours. After the reaction was finished, the solvent was distilled off (at 125°C, 1 kPa or less). Finally, celite was added into the obtained oily compound, the mixture was well mixed, and then filtered for removing an unreacted polyoxyalkylene compound to obtain a target compound (colorless and clear oil, yield: 115.3 g). The number-average molecular weight was Mn = 13786. This compound was a compound represented by the general formula (1), wherein a = 18, b = 5, c = 0, d = 15, e = 9, f = 0, and g = 3.

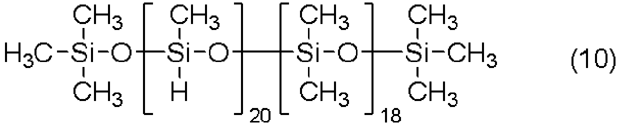

### Example 3

Into a separable flask, an organohydrogenpolysiloxane (100 g, number of moles of hydrosilyl groups: 74.6 mmol) with an average composition represented by the following general formula (11), a cyclic polysiloxane (8.2 g, 26.6 mmol) represented by the general formula (8), and a 3% ethanol solution of chloroplatinic acid (10 mg) were added, and the mixture was stirred at 90°C under a nitrogen gas flow for 1 hour. Thereafter, a polyoxyalkylene compound (22.8 g, number of moles of allyl groups: 47.7 mmol) with an average composition represented by the general formula (9), isopropyl alcohol (30.0 g), and a 3% ethanol solution of chloroplatinic acid (20 mg) were added, and the mixture was further stirred for 5 hours. After the reaction was finished, the solvent was distilled off (at 125°C, 1 kPa or less). Finally, celite was added into the obtained oily compound, the mixture was well mixed, and then filtered for removing an unreacted polyoxyalkylene compound to obtain a target compound (colorless and clear oil, yield: 107.4 g). The number-average molecular weight was Mn = 9021. This compound was a compound represented by the general formula (1), wherein a = 40, b = 3, c = 12, d = 1, e = 9, f = 0, and g = 3.

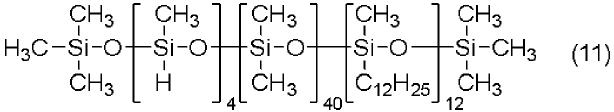

### Comparative Example 1

Into a separable flask, an organohydrogenpolysiloxane (200 g, number of moles of hydrosilyl groups: 0.268 mol) with an average composition represented by the general formula (7), a linear polysiloxane (60.3 g, a number of moles of vinyl groups: 0.107 mol) with an average composition represented by the following general formula (12), and a 3% ethanol solution of chloroplatinic acid (10 mg) were added, and the mixture was stirred at 90°C under a nitrogen gas flow for 1 hour. Thereafter, a polyoxyalkylene compound (80.8 g, number of moles of allyl groups: 0.170 mol) with an average composition represented by the general formula (9), isopropyl alcohol (113.1 g), and a 3% ethanol solution of chloroplatinic acid (20 mg) were added, and the mixture was further stirred for 5 hours. After the reaction was finished, the solvent was distilled off (at 125°C, 1 kPa or less). Finally, celite was added into the obtained oily compound, the mixture was well mixed, and then filtered for removing an unreacted polyoxyalkylene compound to obtain a target compound (colorless and clear oil, yield: 282.2 g). The number-average molecular weight was Mn = 8047.

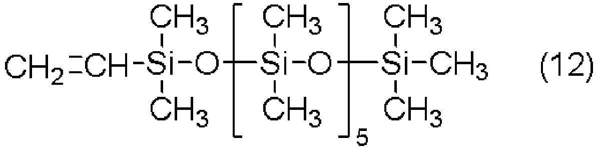

### Comparative Example 2

Into a separable flask, an organohydrogenpolysiloxane (20 g, number of moles of hydrosilyl groups: 0.146 mol) with an average composition represented by the general formula (10), a linear polysiloxane (75.0 g, a number of moles of vinyl groups: 0.0949 mol) with an average composition represented by the general formula (12), and a 3% ethanol solution of chloroplatinic acid (5 mg) were added, and the mixture was stirred at 90°C under a nitrogen gas flow for 1 hour. Thereafter, a polyoxyalkylene compound (25.6 g, number of moles of allyl groups: 0.0534 mol) with an average composition represented by the general formula (9), isopropyl alcohol (35.8 g), and a 3% ethanol solution of chloroplatinic acid (10 mg) were added, and the mixture was further stirred for 5 hours. After the reaction was finished, the solvent was distilled off (at 125°C, 1 kPa or less). Finally, celite was added into the obtained oily compound, the mixture was well mixed, and then filtered for removing an unreacted polyoxyalkylene compound to obtain a target compound (colorless and clear oil, yield: 96.5 g). The number-average molecular weight was Mn = 14534.

### Comparative Example 3

Into a separable flask, an organohydrogenpolysiloxane (100 g, number of moles of hydrosilyl groups: 74.6 mmol) with an average composition represented by the general formula (11), a linear polysiloxane (15.0 g, 48.6 mmol) with an average composition represented by the general formula (12), and a 3% ethanol solution of chloroplatinic acid (10 mg) were added, and the mixture was stirred at 90°C under a nitrogen gas flow for 1 hour. Thereafter, a polyoxyalkylene compound (22.8 g, number of moles of allyl groups: 47.7 mmol) with an average composition represented by the general formula (9), isopropyl alcohol (30.0 g), and a 3% ethanol solution of chloroplatinic acid (20 mg) were added, and the mixture was further stirred for 5 hours. After the reaction was finished, the solvent was distilled off (at 125°C, 1 kPa or less). Finally, celite was added into the obtained oily compound, the mixture was well mixed, and then filtered for removing an unreacted polyoxyalkylene compound to obtain a target compound (colorless and clear oil, yield: 107.4 g). The number-average molecular weight was Mn = 9594.

### Measurement of Interfacial Tension

To confirm how much the compound reduced the interfacial tension, the interfacial tension was measured in the following experience manner. Into a 100-mL beaker, water (25 g) and a polydimethylsiloxane (35 g, KF-96A-2cs: produced by Shin-Etsu Chemical Co., Ltd.) containing each of the surfactants of Examples 1 to 3 and Comparative Examples 1 to 3 at 0.005 wt% were added. An interface formed by leaving to stand was subjected to interfacial tension measurement by using an automatic surface tension meter (DY-300, produced by Kyowa Interface Science Co., Ltd.) with a Wilhelmy method. The measurement was performed twice, immediately after the interface formation and after 4 hours. Table 1 shows the results. The surfactant of the present invention that had the cyclic siloxane chain on the side chain exhibited lower interfacial tension.

**[Table 1]**

| Interfacial tension [mN/m] | Example 1 | Comparative Exmaple 1 | Example 2 | Comparative Exmaple 2 | Example 3 | Comparative Exmaple 3 | Without surfactant |
|---|---|---|---|---|---|---|---|
| immediately after interface formation | 9.31 | 14.65 | 15.84 | 21.32 | 10.54 | 12.06 | 40.24 |
| after 4 hours | 2.96 | 5.44 | 5.74 | 9.54 | 5.81 | 7.34 | - |

Hereinafter, formulation examples of cosmetics containing the surfactant of the present invention will be described. The present invention is not limited by these formulation examples. A blending amount means a blending amount in a blended product.

### Formulation Example 1: Lipstick

| (Component) | | mass (%) |
|---|---|---|
| 1. | Dextrin Palmitate/Ethylhexanoate (INCI) | 9.0 |
| 2. | Triethylhexanoin (INCI) | 22.0 |
| 3. | Bentonite (INCI) | 0.7 |
| 4. | Surfactant described in Example 3 | 1.5 |
| 5. | Cyclopentasiloxane (INCI) | 42.0 |
| 6. | Butylene Glycol (INCI) | 5.0 |
| 7. | Purified water | 19.8 |
| 8. | Coloring pigment | appropriate amount |
| Total | 100.0 | |

### (Producing Method)

A: The component 1, a part of the component 2, and the components 3 to 5 were mixed and dissolved.
B: The component 8 was mixed with the remainder of the component 2, and dispersed with a roller.
C: B) was added into A), and uniformly mixed.
D: The component 6 and the component 7 were mixed and heated.
E: D) was added into C) to be emulsified.

The lipstick obtained as above was confined to be a W/O-type cream lipstick having excellent cosmetic retainability and light extensibility without tackiness and oiliness.

### Formulation Example 2: Eyeshadow

| (Component) | | mass (%) |
|---|---|---|
| 1. | Cyclopentasiloxane (INCI) | 15.0 |
| 2. | Dimethicone (INCI) (Note 1) | 10.0 |
| 3. | Surfactant described in Example 1 (INCI) | 2.0 |
| 4. | Laureth-10 (INCI) | 0.5 |
| 5. | Silicone-treated chromium oxide (Note 2) | 6.2 |
| 6. | Silicone-treated ultramarine blue (Note 2 | 4.0 |
| 7. | Silicone-treated titanium-coated mica (No te 2) | 6.0 |
| 8. | Sodium chloride | 2.0 |
| 9. | Propylene Glycol (INCI) | 8.0 |
| 10. | Antiseptic agent | appropriate amount |
| 11. | Perfume | appropriate amount |
| 12. | Purified water | remainder |
| Total | | 100.00 |

| | | |
|---|---|---|
| (Note 1) KF-96A-6cs, produced by Shin-Etsu Chemical Co., Ltd. (Note 2) Silicone treatment; methylhydrogenpolysiloxane was added at 3% into a powder, and heat-treated. | | |

### (Producing Method)

A: The components 1 to 4 were mixed, the components 5 to 7 were added, and uniformly dispersed.
B: The components 8 to 10 and 12 were uniformly dissolved.
C: Under stirring, B was gradually added into A to be emulsified, and the component 11 was added to obtain the eyeshadow.

The eyeshadow obtained as above was confirmed to provide feel of use having light extensibility without oiliness and powdery feeling, and wetness and freshness, to have good water resistance, water repellency, sweat resistance, and good durability, to hardly cause makeup smudging, and to have excellent stability without change with temperature and time.

### Formulation Example 3: Sunscreen Cream

| (Component) | | mass (%) |
|---|---|---|
| 1. | Cyclopentasiloxane (INCI) | 17.5 |
| 2. | (Acrylates/Dimethicone) Copolymer (INCI) (Note 3) | 12.0 |
| 3. | Triethylhexanoin (INCI) | 5.0 |
| 4. | Ethylhexyl Methoxycinnamate (INCI) | 6.0 |
| 5. | (Dimethicone/(PEG-10/15) Crosspolymer (INCI) (Note 4) | 5.0 |
| 6. | Surfactant described in Example 1 | 1.0 |
| 7. | Zinc oxide fine particles | 20.0 |
| 8. | Sodium chloride | 0.5 |
| 9. | Butylene Glycol (INCI) | 2.0 |
| 10. | Antiseptic agent | appropriate amount |
| 11. | Perfume | appropriate amount |
| 12. | Purified water | remainder |
| Total | | 100.0 |

| | | |
|---|---|---|
| (Note 3) KP-545: produced by Shin-Etsu Chemical Co., Ltd. (Note 4) KSG-210: produced by Shin-Etsu Chemical Co., Ltd. | | |

### (Producing method)

A: The component 2 was added into a part of the component 1 to be uniform, and the component 7 was added and dispersed with a bead mill.
B: The remainder of the component 1 and the components 3 to 6 were mixed, and uniformly mixed.
C: The components 8 to 10 and 12 were mixed and dissolved.
D: C was added into B to be emulsified, and A and the component 11 were added to obtain sunscreen cream.

The sunscreen cream obtained as above was confirmed to have light extensibility without tackiness, excellent adhesion feeling, good settlement, gloss finish, extremely excellent makeup durability, and extreme stability against temperature and time.

### Formulation Example 4: Liquid Foundation

| (Component) | | mass (%) |
|---|---|---|
| 1. | Cyclopentasiloxane (INCI) | 16.0 |
| 2. | Dimethicone (INCI) (Note 1) | 8.0 |
| 3. | Ethylhexyl Methoxycinnamate (INCI) | 3.0 |
| 4. | Hydroxystearic Acid (INCI) | 1.0 |
| 5. | Fluorine-modified silicone (Note 5) | 15.0 |
| 6. | Surfactant described in Example 2 | 5.0 |
| 7. | Polymethylsilsesquioxane (INCI) (Note 6) | 3.0 |
| 8. | Fluorine-compound-treated titanium oxide fine particles (NOTE 7) | 8.0 |
| 9. | Fluorine-compound-treated mica titanium (Note 7) | 1.0 |
| 10. | Fluorine-compound-treated titanium oxide(Note 7) | 5.0 |
| 11. | Fluorine-compound-treated Red Oxide (Note7) | 0.9 |
| 12. | Fluorine-compound-treated yellow iron oxide (Note 7) | 2.0 |
| 13. | Fluorine-compound-treated black iron oxide (Note 7) | 1.0 |
| 14. | Ethanol | 15.0 |
| 15. | Glycerol | 3.0 |
| 16. | Magnesium sulfate | 1.0 |
| 17. | Antiseptic agent | appropriate amount |
| 18. | Perfume | appropriate amount |
| 19. | Purified water | remainder |
| Total | | 100.0 |

| | | |
|---|---|---|
| (Note 1) KF-96A-6cs: produced by Shin-Etsu Chemical Co., Ltd. (Note 5) FL-100-100cs: produced by Shin-Etsu Chemical Co., Ltd. (Note 6) KMP-590: produced by Shin-Etsu Chemical Co., Ltd. (Note 7) Fluorine-compound treatment; 5%-coated with diethanolamine salt of perfluoroalkylethylphosphate. | | |

### (Producing Method)

A: The components 7 to 13 were uniformly mixed.
B: The components 1 to 6 were heated and mixed at 70°C, and A was added and uniformly dispersed and mixed.
C: The components 14 to 17 and 19 were heated at 40°C and gradually added into B to be emulsified, cooled, and the component 18 was added to obtain liquid foundation.

The liquid foundation as above was confirmed to have light extensibility without tackiness, plain and high refresh feeling, and extremely excellent stability without change with temperature and time.

### Formulation Example 5: O/W Hand Cream

| (Component) | | mass (%) |
|---|---|---|
| 1. | (Acrylates/Dimethicone) Copolymer (INCI)(Note 3) | 10.0 |
| 2. | (Acrylates/Stearyl Acrylate/Dimethicone Methacrylate) Copolymer (Note 8) (INCI) | 8.0 |
| 3. | Cetanol (INCI) | 1.0 |
| 4. | Glyceryl Isostearate (INCI) | 5.0 |
| 5. | Stearic Acid (INCI) | 3.0 |
| 6. | Glyceryl Stearate | 1.5 |
| 7. | Surfactant described in Example 3 | 0.7 |
| 8. | Sorbitan Sesquioleate (INCI) | 0.5 |
| 9. | PEG-6 Sorbitan Oleate (INCI) | 1.0 |
| 10. | Sodium hydroxide (1% aqueous solution) | 10.0 |
| 11. | Butylene Glycol (INCI) | 5.0 |
| 12. | Antiseptic agent | appropriate amount |
| 13. | Perfume | appropriate amount |
| 14. | Purified water | remainder |
| Total | | 100.0 |

| | | |
|---|---|---|
| (Note 3) KP-545: produced by Shin-Etsu Chemical Co., Ltd. (Note 8) KP-561: produced by Shin-Etsu Chemical Co., Ltd. | | |

### (Producing Method)

A: The components 1 to 9 were mixed, and dissolved with heating.
B: The components 10 to 12 and 14 were mixed, and heated.
C: B was added into A to be emulsified, cooled, and the component 13 was added to obtain O/W hand cream.

The hand cream obtained as above was confirmed to have light extensibility without tackiness, excellent adhesion feeling, good settlement, gross finish, extremely excellent makeup durability, and extreme stability with temperature and time.

### Formulation Example 6: Milky Lotion

| (Component) | mass (%) |
|---|---|
| 1. Cyclopentasiloxane (INCI) | 15.0 |
| 2. Diphenylsiloxy Phenyl Trimethicone (INCI) (NOTE 9) | 5.0 |
| 3. Squalene (INCI) | 5.0 |
| 4. Pentaerythrityl Tetraethylhexanoate (INCI) | 5.0 |
| 5. Surfactant described in Example 1 | 3.0 |
| 6. Polymethylsilsesquioxane (INCI) (Note 10) | 2.0 |
| 7. Hydrophobized silica (Note 11) | 0. 5 |
| 8. Magnesium Ascorbyl Phosphate | 1.0 |
| 9. Sodium chloride | 1.0 |
| 10. PEG-240 (INCI) | 1.0 |
| 11. Propylene Glycol (INCI) | 8.0 |
| 12. Antiseptic agent | appropriate amount |
| 13. Perfume | appropriate amount |
| 14. Purified water | remainder |
| Total | 100.0 |

| | |
|---|---|
| (Note 9) KF-56A: produced by Shin-Etsu Chemical Co., Ltd. (Note 10) KMP-594: produced by Shin-Etsu Chemical Co., Ltd. (Note 11) AEROSIL R972: produced by NIPPON AEROSIL CO., LTD. | |

### (Producing Method)

A: The components 1 to 5 were uniformly mixed, the components 6 to 7 were added, and uniformly dispersed.
B: The components 8 to 10 were added into the component 14, dissolved, the components 11 and 12 were uniformized and then further added.
C: B was gradually added into A to be emulsified, then cooled, and the component 13 was added to obtain milky lotion.

The milky lotion obtained as above was confirmed to have light extensibility, plain and no tackiness, and excellent usability and stability without change with temperature and time.

### Formulation Example 7: Makeup Remover

| (Component) | | mass (%) |
|---|---|---|
| 1. | Surfactant described in Example 1 | 20.0 |
| 2. | Polysorbate 60 (INCI) | 10.0 |
| 3. | Sorbitol | 10.0 |
| 4. | Carrageenan | 0.5 |
| 5. | Antiseptic agent | appropriate amount |
| 6. | Perfume | appropriate amount |
| 7. | Purified water | remainder |
| Total | | 100.0 |

### (Producing Method)

A: The components 1 to 5 and 7 were added, and uniformly dissolved.
B: The component 6 was added into A to obtain makeup remover.

The makeup remover obtained as above was used to remove foundation with good durability. The makeup remover had good compatibility with the foundation and sebum stain, an extremely good stain removing property, light extensibility in use, yielded no tackiness and plain skin after use, and extremely excellent usability and feel of use. The makeup remover was also confirmed to have excellent stability without change with temperature and time.

The cosmetic containing the surfactant of the present invention provides the cosmetic having light extensibility without oiliness and tackiness, plain feel of use with wetness and freshness, extremely good makeup durability, and extremely excellent stability without change with temperature and time.

### Industrial Applicability

The cosmetic of the present invention has excellent feel of use and good stability with time, and is therefore extremely useful in practice. In addition, the composition of the present invention is the raw material component for the practically used cosmetic, and therefore large industrial usefulness.

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

## Claims

1. A surfactant represented by the following general formula (1), 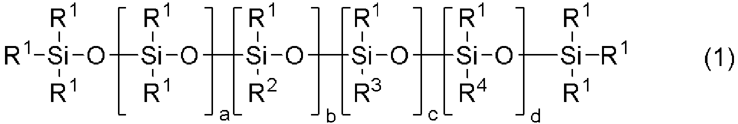 wherein R¹ independently represents a group selected from an alkyl group having 1 to 4 carbon atoms and an aryl group having 6 to 10 carbon atoms, R² independently represents a group represented by the following general formula (2), R³ independently represents an alkyl group having 6 to 20 carbon atoms, R⁴ independently represents a group having a cyclic siloxane structure represented by the following general formula (3), a = 10 to 60, b = 1 to 30, c = 0 to 30, d = 1 to 30, provided that "a", "b", "c", and "d" satisfy a range of a + b + c + d = 20 to 100, and a bonding order of siloxane units in brackets with "a" to "d" may be block or random, 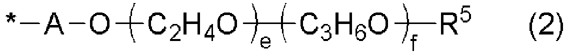 wherein A represents an alkylene group having 2 to 10 carbon atoms, R⁵ represents a hydrogen atom or an alkyl group having 1 to 10 carbon atoms, "e" represents an integer satisfying e = 0 to 100, "f" represents an integer satisfying f = 0 to 100, provided that e + f represents an integer of 2 to 200, a bonding order of alkylene oxide units in brackets with "e" and "f" may be block or random, and "*" represents an attachment bonded to a silicon atom, 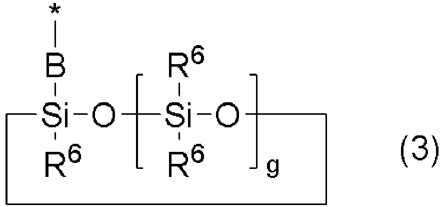 wherein B represents an alkylene group having 2 to 10 carbon atoms, R⁶ independently represents a hydrogen atom or a group selected from an alkyl group having 1 to 4 carbon atoms and an aryl group having 6 to 22 carbon atoms, "g" represents an integer satisfying g = 2 to 5, and "*" represents an attachment bonded to a silicon atom.

2. The surfactant according to claim 1, wherein the surfactant comprises a cyclic siloxane wherein R⁶ represents a methyl group and g = 3 in the general formula (3).

3. A cosmetic, comprising the surfactant according to claim 1.

4. The cosmetic according to claim 3, wherein the cosmetic has an emulsion form.
